(19) Europäisches Patentamt

European Patent Office

Office européen des brevets

(11) EP 0 787 465 B1

## (12) EUROPEAN PATENT SPECIFICATION

(45) Date of publication and mention
of the grant of the patent:
**13.01.1999 Bulletin 1999/02**

(51) Int. Cl.$^6$: **A61B 17/36**

(21) Application number: **96400215.8**

(22) Date of filing: **31.01.1996**

(54) **Cold plasma coagulator**

Kaltplasmakoagulierungsvorrichtung

Appareil de coagulation à plasma froid

(84) Designated Contracting States:
**BE CH DE FR GB LI NL**

(43) Date of publication of application:
**06.08.1997 Bulletin 1997/32**

(73) Proprietor:
**JUMP TECHNOLOGIES LIMITED**
**Hong Kong (HK)**

(72) Inventors:
  • **Soo-In, Kim**
    **Seocho-Ku, Seoul (KR)**
  • **Vladimir, Nikolaevich Lisin**
    **111024 Moscow (RU)**
  • **German, Bekker**
    **Moscow (RU)**
  • **Sergei, Malivanov**
    **Moscow (RU)**

(74) Representative:
**Hirsch, Marc-Roger et al**
**Cabinet Hirsch**
**34 rue de Bassano**
**75008 Paris (FR)**

(56) References cited:
**FR-A- 2 699 785**     **US-A- 3 903 891**
**US-A- 3 938 525**     **US-A- 3 991 764**

## Description

### Field of the Invention

The present invention relates to a novel plasma coagulator, more specifically, to a cold plasma coagulator to clot the blood bleeding from the tissue during surgical operation in an effective and simple manner, by applying unipolar cold plasma.

### Background of the Invention

Various types of blood coagulators have been developed in the art to clot the blood, which minimizes the danger resulting from excessive loss of blood during surgical operation. In particular, plasma coagulators which transform inlet gas to plasma and clot the blood by applying the plasma to the tissue during surgical operation, have been widely used in the clinical surgery and actively studied by the researchers, due to its simple structure and high coagulation efficiency.

For example, USP 3,903,891, USP 3,938,525, USP 3,991,764, USP 4,562,838, USP 4,781,175 and USP 5,207,675 disclose the various types of plasma coagulators in the art.

The plasma coagulators of the prior art are generally classified into two classes, in accordance with their structure, and principle or mode of plasma generation:

One type of plasma coagulators comprise power supply, gas dynamic path and plasmotron. Cylindrical case of the plasmotron has an output channel for plasma, an anode being connected to electrical circuit of power supply, and a cathode being connected to a cylindrical holder installed coaxially with the said case and a rod-like electrode fixed in the end of the holder. The plasma is generated from the plasma forming chamber located between free end of the said electrode and internal sectional face of the output channel, in a form of an arc or glow discharge generated between the anode and the cathode.

The plasma coagulators of the prior art, however, have revealed shortcomings as follows: they essentially requires a large cooler and a heat exchanger to cool off heated head of the plasmotron, because the plasma is formed during a heating-up over several thousand degree of temperature, and the arc generated between the electrodes is very unstable. Further, when the plasma coagulator is practically operated, the shunting of arc arises and the laminar flow of plasma may be disturbed, which finally gives unstable plasma. When the charge of plasma forming gas is low, unstable plasma is also generated and the plasma discharge can be stabilized only by regulating the charge of gas, which results in malfunction of the plasma coagulator. The said limitations of the prior art is presumed to be grounded on the fact that the length of the plasma arc is fixed by a variety of parameters depended on power supply and gas dynamic path, e.g., arc current, charge and pressure of gas, and the diameter of output channel in the plasma coagulator.

Moreover, the plasma coagulators of the prior art have low coagulation efficiency and increased weight and dimension, and they essentially require an extra ballast resistance in order to regulate the parameters of power supply and load, and to compensate for the lowering of Volt-Ampere characteristics of the plasma coagulators.

Another type of plasma coagulators (as described in US-A-3 908 891) comprise high frequency power supply, gas dynamic path and plasmotron. The plasmotron is assembled in a cylindrical case where a high frequency inductor is positioned, and a dielectric tube functioning as a chamber for plasma generation is located on the axis of coil to help the outlet of plasma forming gas. In the plasma coagulator of this type, voltage generated from high frequency generator which creates tens of MHz, is transferred to the inductor, and gas flow in the dielectric tube is heated up by the conductive ring current flow, which finally generates the plasma.

The plasma coagulators of the prior art, however, have the disadvantages as follows: the plasma generated from the plasma coagulator are of very high temperature of 4,000 to 9,000°C and a high frequency current directly conducting to the tissue may be generated from the plasma coagulator, which possibly causes fatal damages to the tissue during surgical operation, and takes long time to coagulate the blood.

Further, the plasma coagulators, in general, have low coagulation efficiency and it is essential to heat the coil up to high temperature by employing high active capacitor; and, therefore, the mass and dimension of power supply naturally increases to provide high electric conductivity during gas flow. Especially, when the plasma coagulators of the prior art are applied to the tissue which bleeds in high volume, e.g., to the liver, it has revealed that the coagulators show very low coagulation efficiency. Moreover, the energy consumption of the coagulators is more than thousands of kV, and the electric shock may be caused when the coagulators are practically used, because the plasma generated therefrom has bipolar characteristics. The plasma coagulators, also, have very complicated structure, which results in increase of the costs for manufacturing. Accordingly, they have been proven to be less satisfactory in the sense that they can not be practically used in the clinical surgery.

### Summary of the Invention

A primary object of the invention is, therefore, to provide a cold plasma coagulator to clot the blood bleeding from the tissue during surgical operation which generates unipolar cold plasma to cause no electric shock, reduces the damages of tissue, requires lower energy consumption, and is of high coagulation efficiency and simple structure to minimize the mass and dimension.

Brief Description of the Drawings

The above object of the present invention will become apparent from the following descriptions given in conjunction with the accompanying drawings, in which:

Figure 1      is a structural diagram of the plasmotron of the cold plasma coagulator;

Figure 2      is an unit-block diagram of the cold plasma coagulator;

Figure 3      is a brief cross-sectional view of the resonance inductor of the cold plasma coagulator;

Figure 4      is a schematic diagram of the plasmotron case of the cold plasma coagulator;

Figure 5      is an equivalent circuit diagram to resonance characteristics of the cold plasma coagulator; and,

Figures 6(a) to 6(d)      are waveform diagrams for the power supply and resonance circuit of the cold plasma coagulator.

Detailed Description of the Invention

The cold plasma coagulator of the present invention comprises high frequency power supply, gas dynamic block and plasmotron. The said power supply comprises rectifier, capacitor storage and voltage inverter. The said plasmotron is assembled in hermetically sealed case in which resonance inductor and dielectric tube of cylindrical cartridge are located coaxially, one end of the dielectric tube being connected to the said gas dynamic block and another end being positioned to eject plasma through output nozzle, and along the axis of the dielectric electrode a needle corona electrode being located, coils of the resonance inductor consisting of low voltage and high voltage sections, the low voltage section of resonance coils being connected to output of the voltage inverter, one pin of the high voltage section being isolated and another one being connected to the needle corona electrode.

In this regard, the high voltage section of resonance inductor is provided with cylindrical multi-layer, its non-isolated pin being connected to internal layer of the resonance coils and the isolated pin being connected to external layer, and the low voltage section being reeled up atop of the high voltage section in one layer.

Further, employments of a fluoroplastic output nozzle, a sealing nipple installed at one end of the output nozzle to connect with the dielectric tube, a cone-shaped plasma forming chamber provided at another end of the nozzle, facilitate the operation of the plasma coagulator. Preferably, the plasmotron case is constructed in a dismountable banana-shaped form, consisting of two symmetric halves. It is also preferred that the resonance inductor is wound on the fluoroplastic body whose internal and external diameters and height cover the ranges fixed by the following ratio:

$$2 < h/D < 3 \qquad 0.3 < d/D < 0.5$$

wherein,

D and d      represent internal and external diameters, respectively; and,

h      represents height of the fluoroplastic body.

The cold plasma coagulator of the invention can generate stable flow of cold plasma even under low charge of plasma forming gas or low current of discharge, which permits decrease of the mass and dimension and efficient operation without applying active capacitor for reactive current.

According to the cold plasma coagulator of the invention, the plasma is excited by the high frequency magnetic field generated from the cavity of resonance coils and by the high frequency induction electrical field from electrode system, and this excitation process does not require additional capacitor for the conductive current.

While the plasma from the prior art coagulator, which is generated by Faraday induction, has bipolar characteristics, the cold plasma from the coagulator of the invention is generated by unipolar induction. Therefore, the currents and potentials for the cold plasma generation obey the law of unipolar induction, and the currents recur to the power supply unit, so the consumption of energy in the plasma coagulator decreases. That is, the plasma coagulator of the invention which generates cold plasma always operates in a form of unipolar induction, so plasma energy can reach to the object through conductive medium. The cold plasma, generated from the plasma coagulator can be activated and accumulated by inductance coils during multi-dimensional parametric resonance process.

A preferred embodiment of the present invention is explained in detail with references on the accompanying drawings, which should not be taken to limit the scope of the present invention.

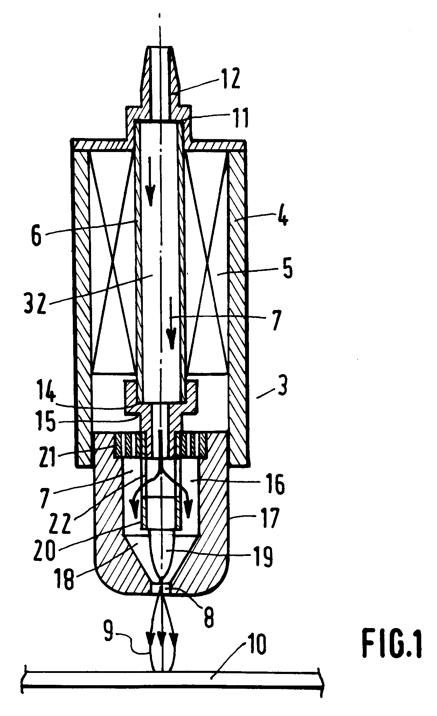

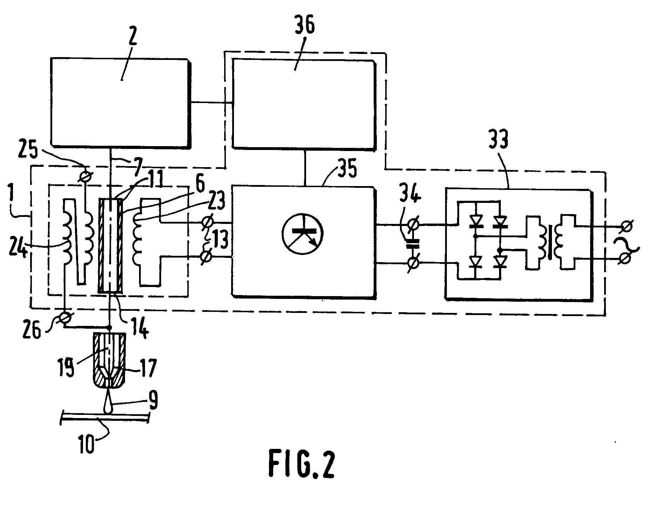

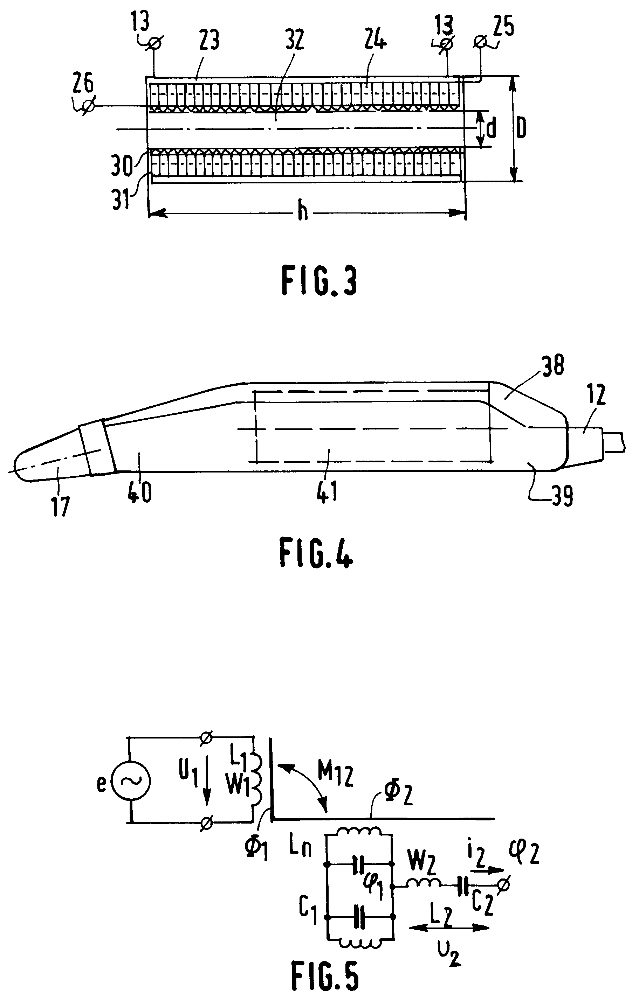

Referring to Figs. 1, 2, and 3, the plasma coagulator of the invention comprises high frequency power supply unit(1), gas dynamic block(2) and plasmotron(3), all of which are assembled in cylindrical case(4) so that resonance inductor(5) and dielectric tube(6) to flow plasma forming gas(7) are located coaxially. The power supply unit comprises rectifier(33), capacitor storage(34) and voltage inverter(35), exciting the resonance coils located in the plasmotron(3) by generating sine wave potential with 30 to 60V of voltage, 50 to 150kHz of frequency and 50 to 100W of power, is constructed to allow the ejection of cold unipolar plasma(9) from output nozzle(8), which coagulates the blood bleeding from the tissue(10). The first end face(11) of the dielectric tube(6) is connected to the gas dynamic block(2) through a plug(12) which also functions as a canal for the power supply entrance(13) to supply resonance coil with power. The second end face(14) of the tube(6) is hermetically connected to the cylindrical cavity(16) of plasma forming electrode(17) through nipple(15) and under the cavity(16), plasma forming chamber(18) is positioned. Along the axis of the chamber(18) is installed needle corona electrode(19), the location of which is fixed by the aid of tubular holder(20) and flange(21). The tubular holder(20) is positioned at the side of radial apertures(22), through which the flow of gas arrives at the plasma forming chamber(18).

The coils of resonance inductor consist of low voltage section(23) and high voltage section(24), as depicted in Fig. 2, one pin(25) of the high voltage section (24) is isolated and another one(26) is connected to the needle corona electrode(19) through the tubular holder(20). In this regard, the high voltage section(24) of resonance coils is provided with cylindrical multi-layered one, whose non-isolated pin(26) is connected to internal layer of the resonance coils and the isolated pin(25) to external layer, respectively; and, the low voltage section(23) is reeled up atop of the high voltage section(24) in one layer of the coils. The resonance coils are assembled on dielectric fluoroplastic body(30) and isolated each other by the aid of thin-layer film isolator(31). The dielectric tube(6) is installed in the cylindrical cavity(32) of the coils.

The power supply unit(1) comprises low voltage block transformer-rectifier(33), capacitor storage(34) and voltage inverter(35). The voltage inverter(35) generates sine wave signal of designated resonance frequency. Control block(36) regulates gas pressure through the gas dynamic block(2) and controls voltage of the inverter(35) and tunes resonance frequency of the inverter. The inverter(35) transforms DC voltage of 25 to 35V into the frequency with which monovibrator can work.

The capacity range of the plasmotron(3) of the invention is about 50 to 100$B_T$, the diameter of the output nozzle(8) is less than about 1mm, and the flow velocity of argon employed as a plasma forming gas is about 2 to 6L/min.

Since the coagulation of blood by the cold plasma coagulator of the invention is achieved in a form of corona discharge or high voltage glow discharge, the excitation mechanism, concentration and temperature of the plasma would be varied; however, isolation or second electrode, and conduction of current through the biological object would not be required, grounded on the fact that the generation mechanism follows unipolar induction.

Referring to Fig. 4, the plasmotron case is designed, for the convenience during surgical use, as a banana-shaped form assembled from two halves(38),(39). On the narrow part(40) of the case, the dielectric plasma forming electrode(17) is connected, and at the rear side of the case, the plug(12) for conducting and supplying the gas to resonance coils located in cylindrical cavity(41) is positioned. The banana-shaped plasmotron case in an assembled form facilitates convenient use of the plasma coagulator. Further, since the dielectric electrode(17) is also dismountable, the plasma nozzle with various output diameter would be employed in the plasma coagulator of the invention.

The operation mode of the cold plasma coagulator of the invention is described in more detail with references on the accompanying drawings.

Before operating the plasma coagulator, gas dynamic block(2) is connected to the plasma forming gas(helium or argon) which has fixed flow velocity level of 2 to 6L/min, and the power supply of coagulator switches on. Gas flow from the block(2) which comprises cylinder, controller and storage unit, passesthrough dielectric tube(6) and intersects the central cavity(32) of resonance coils(5), where the magnetic interaction of gas flow and of high frequency magnetic field happens. Though the ionization of the gas would not be achieved by the magnetic interaction, the gas molecules in the flowing gas are initially activated to increase the plasma generation efficiency of the coagulator.

The gas flow in which reactive magnetic conductivity is increased, arrives at the plasma forming chamber(18) in dielectric electrode(17) through nipple(15) and becomes focused at the output nozzle(8) in the dielectric electrode(17) by the formation of signal-deviation corona discharge on needle electrode(19), which finally results in the generation of cold plasma of about 30°C.

As clearly expounded above, because the cold plasma of about 30°C is generated from the coagulator of the present invention, the damages to the tissue which frequently arises from the coagulator of the prior art, may be minimized.

When the focused flow of cold plasma(9) arrives at the object(10) such as the tissue under surgical operation which functions as a kind of conductive material, coagulation of the blood is carried out. In this regard, plasma forming gas(7) interacts with magnetic field and further interacts with electric field at the plasma forming chamber(18).

The resonance characteristics of the coils of resonance inductor(5) are shown briefly in Figs. 3, 4, and 5. In particular, the induction excitation in the low voltage and high voltage sections(23),(24) and the gas flow in central cavity(32) and plasma forming chamber(18) are shown as an equivalent circuit diagram in Fig. 5. Referring to Fig. 5 showing the equivalent circuit diagram of resonance induction coils(5), e represents alternating voltage from inverter(35), and $u_1$, $w_1$, and $L_1$ represent

voltage, number of turns, and inductance for the coils of low voltage section(23), respectively. In the equivalent circuit diagram, the flow of plasma forming gas is represented as the parallel connection $L_n$-$C_n$: wherein, $L_n$ represents the equivalent inductance of plasma, $C_n$ represents the equivalent capacitor of plasma and n represents number of layers of coil.

The mechanism of plasma generation from the coagulator of the invention is defined by the following two emission factors: an emission of charged particles from needle electrode(19) axisymmetrically located in plasma forming chamber(18); and, an emission of the pressurized gas such as helium or argon in the chamber(18). The plasma flow(9) possesses low heat capacity due to gas excitation consisting of double stages. In this regard, the plasma forming gas(7) is initially excited by the high frequency magnetic field in the cylindrical cavity(32) at the stage referred-to as "parallel parametric resonance". In Fig. 5, the parallel parametric resonance of circuit is shown as an equivalent circuit diagram, including the $L_n$-$C_n$ element.

The gas flow is further excited by the high frequency-reactive electric field in the chamber(18), which is shown in the equivalent circuit diagram of Fig. 5 as $L_2$-$C_2$, being connected in series to form a serial resonance circuit. The high inner resistance of the parallel resonance circuit limits total arc current of the plasma generated from the output nozzle(8) of the plasmotron, which permits stable plasma injection, lowers the temperature of the plasma being ejected, and improves the blood coagulation efficiency of the plasma.

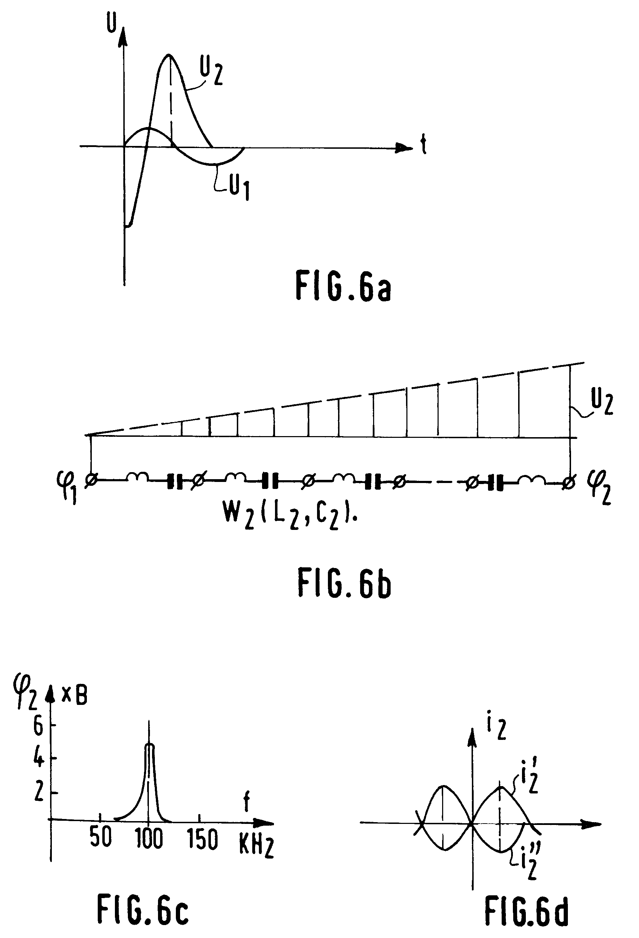

In addition, the plasma has new properties, that is, it has unipolar characteristics which cause no damage and no electric shock to the tissue; and free reactive current $i_2 (=i_2' + i_2'')$ in the parallel resonance circuit(see: Fig. 6(d)) functions as a carrier of unipolar charges.

In Fig. 5, the high voltage section(24) is depicted as a serial resonance circuit of $L_2$-$C_2$ (wherein, the number of turns is represented as $w_2$, the current $i_2$, the voltage $u_2$, and the inductance $L_2$, respectively). The potential for the isolated pin of high voltage section(24) is represented as $\varphi_1$, and the potential of another pin $\varphi_2$. $\Phi_1$ and $\Phi_2$ represent the magnetic flows of low voltage and high voltage sections, respectively, and $M_{12}$ represents the mutual inductance for said sections.

Referring to Fig. 6(a), the main feature of the coagulator under operation is shown as a parametric resonance based on the orthogonal shift of magnetic flows of $\Phi_1$ and $\Phi_2$(or voltages of $u_1$ and $u_2$ of two coil sections). In the resonance zone, the voltage $u_2$ is n times the transformation factor, which is determined by the equation of $Ktr = w_2/w_1$ at the resonance circuit $L_2$-$C_2$. In this regard, voltage $u_2$ may be represented as the equation(1):

$$u_2 = u_1 \cdot Ktr \cdot n \qquad (1)$$

Further, the resonance is accompanied by "polarization" of high voltage section(24), and the field distribution of potential thereby can be depicted as Fig. 6(b). That is, one end of connection is distributed to have a potential of $\varphi_1 = 0$, while another end of connection has a potential of $\varphi_2 = \varphi_{max}$. Especially, the resonance circuit of Fig. 5 resonates at the given frequency of $f_0 = 100kHz$ as shown in Fig. 6(c). If a parametric connection of resonance circuit exists, it will follow the principle of electromagnetic induction represented as the equations(2) and (3):

$$d(LI)/dt = L \cdot dI/dt + I \cdot dL/dt \qquad (2)$$

$$= L \cdot I \qquad (3)$$

The pulse voltage $u_2$ of resonance frequency $w_0$ is generated from the circuit of high voltage section(24) of inductor, and $u_2$ may be represented as the equation(4):

$$u_2 = i_2 \cdot dL/dt \qquad (4)$$

The parameters in the circuit of orthogonal flow $\Phi_2$ can be determined by Mateu equations. If $L_{20}$ designates the average value of inductance for high voltage section(24), $L_2(t)$ may be represented as the equations(5) and (6):

$$L_2(t) = L_{20}/(1 - m \cdot \cos 2\omega_0 t) \qquad (5)$$

wherein,

m     represents the modulation factor of inductance $L_2$.

$$m = 2/\omega_1 \cdot C_{20} \cdot \rho_n \qquad (6)$$

wherein,

$\omega_1$     represents the frequency of output voltage e;
$C_{20}$     is the average value of capacitor for high voltage section(24) under parametric resonance state; and,
$\rho_n$     represents wave resistance of the load(plasma).

The condition of excitation for the parametric resonance may be determined by the equation(7):

$$\omega_0 = \omega_1 \qquad m > 2Q \qquad Q = \omega_1 \cdot C_{20} \cdot \rho_n \qquad (7)$$

$$\omega_0^2 = 1/L_{20} \cdot C_{20}$$

wherein,

Q     is the factor of merit for resonance circuit.

At this condition, the gas flow(7) interacts with magnetic flow $\Phi_2$ through passing the dielectric tube(6) with the velocity V, and its change per time may be determined as a function of $L_2(t)$ as shown in Fig. 6(d), if the current $i_2$ consists of two counterphase harmonics:

$$\Phi_2 = i_2 \cdot L_2(t) \qquad (8)$$

Practically, the high voltage section(24) functions on the active capacitor in the form of idle course (its circuit is disconnected galvanically). In this regard, the pin(25) of the section, possessing the potential of $\varphi_1 = 0$, is not grounded.

In the area of central cavity(32), the scalar magnetic field $H_0$ influences on the gas, as parametric induction is manifested with frequency $\omega_0$. Pointing flow on the axis of resonance coils is defined as:

$$\Pi_H = H_0 \times H \qquad (9)$$

wherein,

H      represents the vectorial magnetic field created by low voltage section(where, the number of turns is $w_1$); and,

$H_0$     represents the scalar magnetic field generated by high voltage section(where, the number of turns is $w_2$).

The electrical portion of Pointing flow arrives into the gas from corona electrode(19) located in plasma forming chamber(18):

$$\Pi_E = E_0 \times E \qquad (10)$$

wherein,

$E_0$     is the scalar electrical field generated by capacitor $C_2$; and,

E      is the vectorial electrical field generated by section $w_2$.

Specifically, the scalar field is generated when the potential of non-isolated pin(26) has a value of $\varphi_1 = 0$ and the potential of isolated pin(25) has the maximum value of 5 to 10kV. Owing to the scalar field $E_0$ and $H_0$, the cold unipolar plasma is generated from the coagulator, that is, the characteristics of resonance inductance coils are changed by the scalar field $E_0$ and $H_0$, and the bipolar electromagnetic induction is transformed into Tesla unipolar current. In this regard, the maximum value of factor of merit for the coils under the parametric resonance state, which is the excitation state of scalar field of $E_0$ and $H_0$, may be achieved by constructing the structure of the fluoroplastic body as followings:

$$2 < h/D < 3 \qquad 0.3 < d/D < 0.5$$

wherein,

D and d      represent internal and external diameter, respectively; and,

h          represents height of the fluoroplastic body.

As far as the resonance circuit gives rise to any leakage, the excitation of plasma may be executed without Joule's heat transfer from the electrical circuit in gas flow. Therefore, the phenomenon of cold plasma ejection can be stipulated based on new mechanism of carrying the energy of scalar electrical and magnetic field in a flow of neutral gas such as helium or argon. The contraction and focusing of plasma flow(9) can be achieved by controlling the aperture diameter of output nozzle(8), as well as by controlling the size of chamber(18) and working pressure of the gas.

As shown in Fig. 6(b), the spontaneous polarization of section $w_2$ transfers the characteristics of coaxial quarter wave resonator to the coil shown in Fig. 3 on scalar fields $E_0$ and $H_0$, then considerably increases the factor of merit Q, which already does not depend on the active resistance of winding, of the resonance circuit. The process of plasma excitation is initiated by polarized currents of section $w_2$ and is executed on a level of free reactive capacitor. In this regard, high potential may be directly formed in the plasmotron by the output for the parametric resonance, and the optimum potential formed at section $w_2$ is 5 to 10kV.

In contrast to the coagulator of the prior art which requires high level of power in generating conductive currents, the cold plasma coagulator of the present invention substantially minimizes the power consumption, grounded that the plasma is activated by reactive currents. Moreover, in contrast to the coagulator of the prior art for which the frequency of tens of MHz has been required, the optimum frequency for the cold plasma coagulator of the invention requires about 100 to 150 kHz, which permits the coagulator to employ the transistor inverter or amplifier with output voltage of 30 to 60V as the power supply unit, so it may be achieved that the mass and dimensional parameters of power supply unit would be considerably reduced to provide a portable plasma coagulator and that the plasma coagulator would be manufactured in an economical manner.

As clearly demonstrated and illustrated as above, the cold plasma coagulator of the present invention has high coagulation efficiency and can generates unipolar cold plasma to cause no electric shock and to minimize the damages of tissue during surgical operation, requiring lower energy consumption, and has simple structure to reduce the mass and dimension.

**Claims**

1.   A cold plasma coagulator which comprises high frequency power supply(1), gas dynamic block(2) and plasmotron(3), the said power supply(1) comprising rectifier(33), capacitor storage(34) and voltage inverter(35), the said plasmotron(3) being assembled in hermetically sealed case(4) and in which resonance inductor(5) and dielectric tube(6) are located coaxially, one end of the dielectric tube(6) being connected to the said gas dynamic block(2), another end being positioned to eject plasma through output nozzle(8), and along the axis of a dielectric electrode(17) a needle corona electrode(19) being located, the coils of the resonance inductor(5) consisting of low voltage and high voltage sections(23),(24), the low voltage section(23) of resonance coils being connected to output of the voltage inverter(35), one pin(25) of the high voltage section(24) being isolated and another one(26) being connected to the needle corona electrode(19).

2. The cold plasma coagulator of claim 1, wherein the high voltage section(24) of resonance inductor(5) is provided with cylindrical multi-layer, its non-isolated pin(26) being connected to internal layer of the resonance coils, the isolated pin(25) being connected to external layer, and the low voltage section(23) being reeled up atop of the high voltage section(24) in one layer.

3. The cold plasma coagulator of claim 1, wherein the resonance inductor(5) is wound on the fluoroplastic body(30) whose the internal and external diameters and height cover the ranges fixed by the following ratio:

$$2 < h/D < 3 \qquad 0.3 < d/D < 0.5$$

wherein,

D and d    represent internal and external diameters, respectively; and,
h    represents height of the fluoroplastic body.

4. The cold plasma coagulator of claim 1, wherein the plasmotron case(4) is a dismountable banana-shaped form consisting of two symmetric halves(38),(39), on the narrow part(40) of the case(4), dielectric electrode (17) being connected.

## Patentansprüche

1. Eine Kaltplasma-Koagulierungsvorrichtung, umfassend ein Hochfrequenz-Netzgerät (1), einen gasdynamischen Block (2), ein Plasmotron (3), wobei das Netzgerät (1) einen Gleichrichter (33), einen kapazitiven Speicher (34) und Wechselrichter (35) aufweist, wobei das Plasmotron in einem luftdicht verschlossenen Gehäuse zusammengesetzt ist und in dem ein Resonarizinduktor (5) und eine dielektrische Röhre (6) koaxial angeordnet sind, wobei ein Ende der dielektrischen Röhre (6) mit dem gasdynamischen Block verbunden ist (2) und das andere Ende so angeordnet ist, daß es durch eine Ausgangsdüse (8) Plasma ausstoßen kann, eine Nadel-Koronaelektrode (10) entlang der Achse einer dielektrischen Elektrode (17) angeordnet ist, die Resonanzinduktorspulen aus Niederspannung- und Hochspannungsbereichen (23, 24) bestehen, die Niederspannungsbereiche (23) der Resonanzspulen mit dem Ausgang des Wechselrichter (35) verbunden sind, ein Stift (25) des Hochspannungsbereichs (24) isoliert ist und ein anderer Stift(26) mit der Nadel-Koronaelektrode (19) verbunden ist.

2. Die Kaltplasma-Koagulierungsvorrichtung gemäß Anspruch 1, worin der Hochspannungsbereich (24) des Resonanzinduktors (5) zylindrische Mehrschichten aufweist, wobei sein unisolierter Stift (26) mit der inneren Schicht der Resonanzspulen verbunden ist und der isolierte Stift (25) mit der äußeren Schicht verbunden ist, und in einer Schicht der Niederspannungsbereich (23) über die Hochspannungsbereich (24) gewunden ist.

3. Die Kaltplasma-Koagulierungsvorrichtung gemäß Anspruch 1, worin der Resonarizinduktor (5) auf einen Körper (30) aus fluoriertem Kunststoff gewickelt ist, dessen innerer und äußerer Durchmesser und Höhe in den Bereichen liegen, die durch die folgenden Verhältnisse begrenzt sind:

$$2 < h/D < 3 \qquad 0,3 < d/D < 0,5$$

worin

D und d    jeweils für den inneren und äußeren Durchmesser stehen; und,
h    für die Höhe des Körpers aus fluoriertem Kunststoff steht.

4. Die Kaltplasma-Koagulierungsvorrichtung gemäß Anspruch 1, worin das Plasmotrongehäuse (4) zerlegbar ist und die Form einer Banane hat, bestehend aus zwei symmetrischen Hälften (38, 39), wobei die dielektrische Elektrode (17) an den schmalen Teil (40) des Gehäuses (4) angeschloßen ist.

## Revendications

1. Un dispositif de coagulation de plasma à froid qui comprend une alimentation électrique à haute fréquence (1), un bloc dynamique à gaz (2) et un plasmotron (3), ladite alimentation électrique (1) comprenant un redresseur (33), un condensateur de stockage (34) et un onduleur (35), ledit plasmotron (3) étant assemblé dans un boîtier (4) hermétiquement scellé et dans lequel sont disposés coaxialement un inducteur de résonance (5) et un tube diélectrique (6), une extrémité du tube diélectrique (6) étant reliée audit bloc dynamique à gaz (2), l'autre extrémité étant positionnée pour éjecter le plasma à travers une buse de sortie (8), et une électrode corona sous forme d'aiguille (19) étant disposée le long de l'axe d'une électrode diélectrique (17), les bobines de l'inducteur de résonance (5) se composant de parties (23),(24) à basse tension et à haute tension, la partie à basse tension (23) des bobines de résonance étant reliée à la sortie de l'onduleur de tension (35), une broche (25) de la partie (24) à haute tension étant isolée et une autre broche (26) étant reliée à l'électrode corona sous forme d'aiguille (19).

2. Le dispositif de coagulation de plasma à froid selon la revendication 1, dans lequel la partie à haute tension (24) de l'inducteur à résonance (5) est munie d'une multicouche cylindrique, sa broche non isolée (26) étant reliée à la couche inférieur des bobines de résonance, la broche isolée (25) étant reliée de la couche extérieure, et la partie à haute tension et la partie à basse tension (23) étant enroulées au-dessus de la partie à haute tension (24) en une seule couche.

3. Le dispositif de coagulation de plasma à froid selon la revendication 1, dans lequel l'inducteur de résonance (5) est bobiné sur le corps fluoroplastique (39) dont les diamètres intérieur et extérieur et la hauteur sont situés dans des gammes déterminées par les relations suivantes :

$$2 < h/D < 3 \qquad 0,3 < d/D < 0,5$$

dans lesquelles :

D et d représentent respectivement les diamètres intérieur et extérieur ; et
h représente la hauteur du corps fluoroplastique.

4. Le dispositif de coagulation de plasma à froid selon la revendication 1, dans lequel le boîtier de plasmotron (4) est profilé démontable en forme de banane se composant de deux moitiés symétriques (38), (39), l'électrode diélectrique (17) étant reliée à la partie étroite (40) du boîtier (4).

FIG.1

EP 0 787 465 B1

FIG.2

10

FIG.3

FIG.4

FIG.5

FIG.6a

$W_2(L_2, C_2)$.

FIG.6b

FIG.6c

FIG.6d